(19) Europäisches Patentamt / European Patent Office / Office européen des brevets

(11) **EP 3 831 543 B1**

(12) **EUROPEAN PATENT SPECIFICATION**

(45) Date of publication and mention
of the grant of the patent:
**15.03.2023 Bulletin 2023/11**

(21) Application number: **19212992.2**

(22) Date of filing: **02.12.2019**

(51) International Patent Classification (IPC):
**B25J 9/10** (2006.01)    **A61B 34/30** (2016.01)
**B25J 18/00** (2006.01)    **B25J 3/02** (2006.01)
**A61B 90/50** (2016.01)

(52) Cooperative Patent Classification (CPC):
**B25J 9/1065; A61B 34/30; A61B 90/50; B25J 3/02;
B25J 18/007;** A61B 2090/506

(54) **REMOTE CENTRE OF MOTION MECHANISM**

MECHANISMUS MIT FERNBEWEGUNGSPUNKT

MÉCANISME DE CENTRE DISTANT DE MOUVEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**09.06.2021 Bulletin 2021/23**

(73) Proprietor: **Katholieke Universiteit Leuven
KU Leuven Research & Development
3000 Leuven (BE)**

(72) Inventors:
• **GIJBELS, Andy
3010 Kessel-Lo (BE)**
• **SMITS, Jonas
3360 Korbeek-Lo (BE)**

(74) Representative: **AWA Benelux
Avenue Josse Goffin 158
1082 Bruxelles (BE)**

(56) References cited:
EP-A1- 3 556 520        EP-A2- 2 736 680
US-A1- 2015 351 857

• GIJBELS A ET AL: "Design and realisation of a novel robotic manipulator for retinal surgery", 2013 IEEE/RSJ INTERNATIONAL CONFERENCE ON INTELLIGENT ROBOTS AND SYSTEMS, IEEE, 3 November 2013 (2013-11-03), pages 3598-3603, XP032537207, ISSN: 2153-0858, DOI: 10.1109/IROS.2013.6696869 [retrieved on 2013-12-27]

• SMITS JONAS ET AL: "Setup and Method for Remote Center of Motion Positioning Guidance During Robot-Assisted Surgery", 2019 IEEE/RSJ INTERNATIONAL CONFERENCE ON INTELLIGENT ROBOTS AND SYSTEMS (IROS), IEEE, 3 November 2019 (2019-11-03), pages 1315-1322, XP033695865, DOI: 10.1109/IROS40897.2019.8968288 [retrieved on 2020-01-23]

**Description**

**Technical field**

The present invention is related to apparatuses, such as manipulators or robots allowing to move a tool, such as a surgical instrument, about a remote centre of motion.

**Background art**

One remote centre of motion mechanism is known from US 2015/0351857. The mechanism comprises a proximal link moving about a local centre of motion, which is copied to a distal link moving identically to the proximal link about a remote centre of motion. The motion of the proximal link can be copied to the distal link by means of e.g. a parallelogram linkage system interconnecting the proximal link and the distal link. Other equivalent systems are possible as well, such as a pulley and belt system.

One problem often encountered in such remote centre of motion manipulators is that the space available for mounting a tool at the end effector so as to be coaxial with the remote centre of motion is limited. This is due to the fact that the tool must be mounted in proximity of the distal link. Often, this requires custom-made tools to be developed in order to cope with the space requirements.

Another problem often encountered with remote centre of motion manipulators, in particular in the medical field, is that the distance between the local centre of motion and the remote centre of motion must be sufficiently large, in order to prevent that motion of the proximal link is hindered by the patient, or even to prevent that the motion of the proximal link would cause injury to the patient. The proximal link indeed moves about and through the local centre of motion, with a significant downwards motion component and appropriate protection must be provided to prevent hitting the patient or the operating table. Furthermore, a too large distance between the local and remote centres of motion degrades system's stiffness leading to more heavy linkages and manipulators, which in turn are more difficult to manipulate.

Yet another ongoing problem is the continuing need to render the system less bulky, particularly in view of avoiding collisions with other manipulators which would be required on site. Many minimally invasive surgical procedures require multiple incisions to be made, and multiple surgical tools to be inserted in the patient. In addition, often the surgeon needs space to reach with his own hands to the surgical tool. Therefore, the manipulators should be made as compact as possible.

**Summary of the invention**

It is an object of the present invention to overcome one or more of the above problems encountered in the prior art.

According to aspects of the invention, there is therefore provided an apparatus for generating motion around a remote centre of motion as set out in the appended claims. An apparatus for generating motion around a remote centre of motion comprises a base link, a first motion mechanism and a tool holder. A local centre of motion is provided on the base link, and is advantageously fixed to it. The first motion mechanism comprises a proximal link and a distal link coupled to the proximal link, e.g. through a first intermediate link pivotally interconnecting the proximal link and the distal link. The proximal link is coupled to the local centre of motion through a revolute joint and a sliding joint allowing the proximal link to translate relative to the local centre of motion and to rotate about (an axis intersecting) the local centre of motion. The first motion mechanism is configured to copy motion of the proximal link relative to the local centre of motion to an identical motion of the distal link relative to the remote centre of motion. A tool holder is fixed to the distal link. The tool holder defines a tool axis intersecting the remote centre of motion in all positions of the tool holder.

In a first aspect, the distal link defines a first orientation which is inclined relative to the tool axis. The first orientation is advantageously arranged at an angle between 1° and 89° relative to the tool axis, preferably between 5° and 60°, more preferably between 10° and 45°, still more preferably between 25° and 35°. The first orientation can be defined by at least a segment of the distal link which is parallel to at least a segment of the proximal link.

By providing features of the first aspect, the tool axis is made to diverge from the distal link in a direction away from the remote centre of motion. This increases the available mounting space of tools on the tool holder.

Advantageously a connecting link rigidly connects the distal link to the tool holder. The connecting link is advantageously arranged transverse to the tool axis. The connecting link further increases the space between the distal link and the tool holder, which further increases the mounting space.

In a second aspect, the sliding joint defines an axis of translation, e.g. of the proximal link relative to the revolute joint. The sliding joint can e.g. comprise a guide fixed to the revolute joint and a slider fixed to the proximal link. The guide and the proximal link are advantageously arranged at a same side of an axis defined by the local and remote centres of motion, in particular a same side of a reference plane which is orthogonal to a plane of motion of the first motion mechanism, in particular orthogonal to a plane of motion of the sliding joint and which comprises the local and remote centres of motion. Advantageously a translation axis of the sliding joint, e.g. an axis of the guide, is parallel to the tool axis.

By providing features of the second aspect, the motion of the proximal link is completely kept within the perimeter of the other linkages of the apparatus, and protruding linkages which may cause injury to a patient or which may cause collision with other devices are eliminated.

In a third aspect, a second motion mechanism assists the first motion mechanism in effecting an advantageously planar motion. The second motion mechanism can e.g. comprise or consist of a double parallelogram linkage system or equivalent, and is coupled between (a segment of) the base link and the first motion mechanism. Particularly, the second motion mechanism comprises a link fixed to the first intermediate link. The second motion mechanism can be configured to maintain an orientation of the first intermediate link, in particular, to maintain the first intermediate link parallel to an axis through the local and remote centres of motion. The second motion mechanism is advantageously coupled to a segment of the base link which is offset from the axis through the local and remote centres of motion, and which is advantageously parallel to the axis. The segment of the base link is advantageously offset to a side of the axis opposite a side where the proximal link is provided.

By providing features of the third aspect, in particular by coupling the second motion mechanism to a segment of the base link offset from the axis through the local and remote centres of motion, the bulkiness of the linkage system (both the first and the second motion mechanisms) can be reduced, leaving more space for the surgeon, and for other manipulators which may be mounted in the vicinity.

## Brief description of the figures

Aspects of the invention will now be described in more detail with reference to the appended drawings, wherein same reference numerals illustrate same features.

Figure 1 is a schematic of a first apparatus according to the first aspect of the invention comprising a tool holder;
Figure 2 is a schematic of a second apparatus according to the first aspect of the invention, in which the tool holder is spaced apart from a distal link;
Figure 3a is a schematic of a third apparatus according to the second aspect of the invention, in which the translational joint is located at point D of the proximal link;
Figure 3b is a schematic of a modified third apparatus according to the second aspect of the invention, in which the translational joint is located beyond point D of the proximal link;
Figure 4 is a schematic of a fourth apparatus according to the third aspect, in which a part of a base link is offset;
Figure 5 is a schematic of a fifth apparatus incorporating features of the first, second, and third aspects;
Figure 6 is a schematic of a sixth apparatus incorporating features of the first, second, and third aspects and including a pivotally connected support;
Figure 7 is a further schematic of the sixth apparatus illustrating various parameters of the apparatus;
Figure 8 is a schematic of a planar loop of the sixth apparatus.

## Description of embodiments

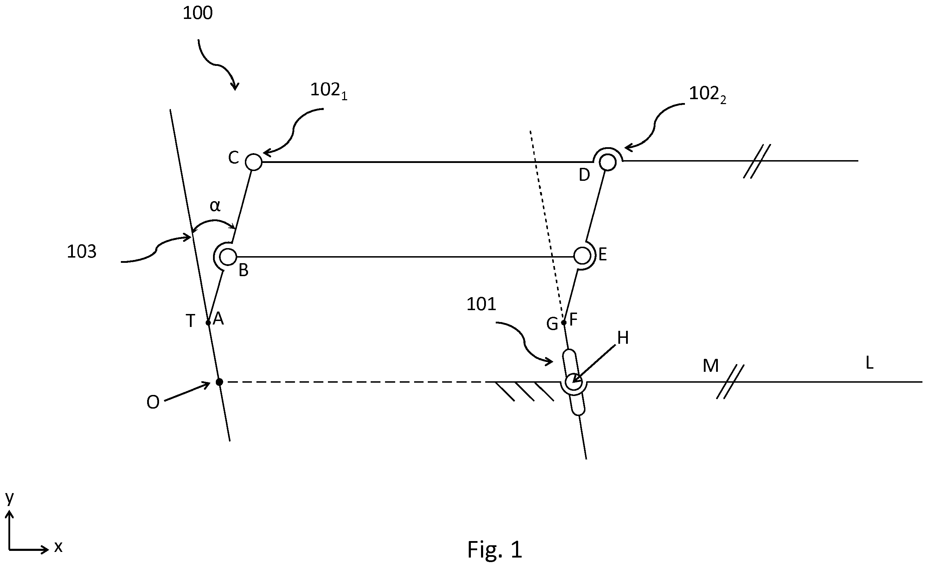

Referring to Figure 1, a first apparatus 100 according to embodiments of the present invention is shown in a side view. The first apparatus 100 comprises a base link HML. The base link HML is a rigid link, for example a rigid bar. The base link HML comprises a local centre of motion H. The apparatus 100 further comprises a first motion mechanism comprising a proximal link DEF, a distal link ABC, and a first intermediate link CD. The first intermediate link CD pivotally interconnects the proximal link DEF and the distal link ABC, for example via rotational couplings $102_1$, $102_2$ at points C and D respectively. The pivotal connection can be implemented, for example, using a shaft in combination with a rotational bearing.

The proximal link DEF is coupled to the local centre of motion H through a revolute and sliding joint 101 which allows both rotation about the local centre of motion H (in the plane of the figure) and translation relative to the local centre of motion H. The sliding joint can e.g. comprise a guide fixed to the revolute joint and a slider fixed to the proximal link. The joint 101 is coupled to the proximal link DEF at point G, which in apparatus 100 coincides with point F. In the first apparatus 100, joint 101 is a combination of a prismatic joint with a rotational joint.

The first motion mechanism is configured to copy motion of the proximal link DEF relative to the local centre of motion H to an identical motion of the distal link ABC relative to the remote centre of motion O. This can be achieved, for example, via the parallelogram BCDE, where the link between points B and E is be a rigid link. Alternatively, a parallel belt drive, or any other suitable mechanical means for copying the motion which is a mechanical equivalent of a parallelogram, may be provided. Links ABC and DEF are rigid links.

The revolute and sliding joint 101 in combination with the first motion mechanism allow the following degrees of freedom to be transferred from the local centre of motion H to the remote centre of motion O: a first degree of freedom θ being a rotational degree of freedom about the remote centre of motion O and perpendicular to the x and y directions; a second degree of freedom R being a translational degree of freedom through the remote centre of motion O and located in the x-y plane. Optional further degrees of freedom can be provided by a rotational connection of the base link HML to a support, as will be described in further detail hereinafter.

The apparatus further comprises a tool holder 103 fixed to the distal link ABC. The tool holder is suitable for coupling to one or more tools, for example surgical tools. The tool holder 103 defines a tool axis OT which extends through the remote centre of motion O. The distal link ABC defines a first orientation which is inclined relative to the tool axis OT. The inclination of the distal link ABC with respect to the tool axis OT is shown as angle α. The angle α is greater than 0° and less than 90° and is preferably between 5° and 60°, more preferably between 25° and 35°. For example, one preferred value of α is 30°.

By providing the tool axis OT at an inclination with respect to the distal link ABC and in a direction away from the remote centre of motion H, more space is provided in the region of the tool axis. This allows easier mounting and removal of tools from the tool holder 3. In surgical applications, this has particular advantages, as the sterilisation of the apparatus and/or any tools used with the apparatus is made easier. In addition, the increase in space in the region of the tool axis allows an operator to more easily access and manipulate a tool coupled to the tool holder 103 and provides more space for such manipulation. This may be desirable for example in a cooperative surgery situation wherein the tool can be manipulated by the operator indirectly via the apparatus, where manipulation of the local centre of motion is transferred to the remote centre of motion, or can be manipulated directly by the operator by grasping the tool itself. Both direct and indirect manipulation may occur, for example the local centre of motion may be manipulated by a driving element which is configured to reduce a tremor of the operator while the operator directly grasps and manipulates the tool at the remote centre of motion.

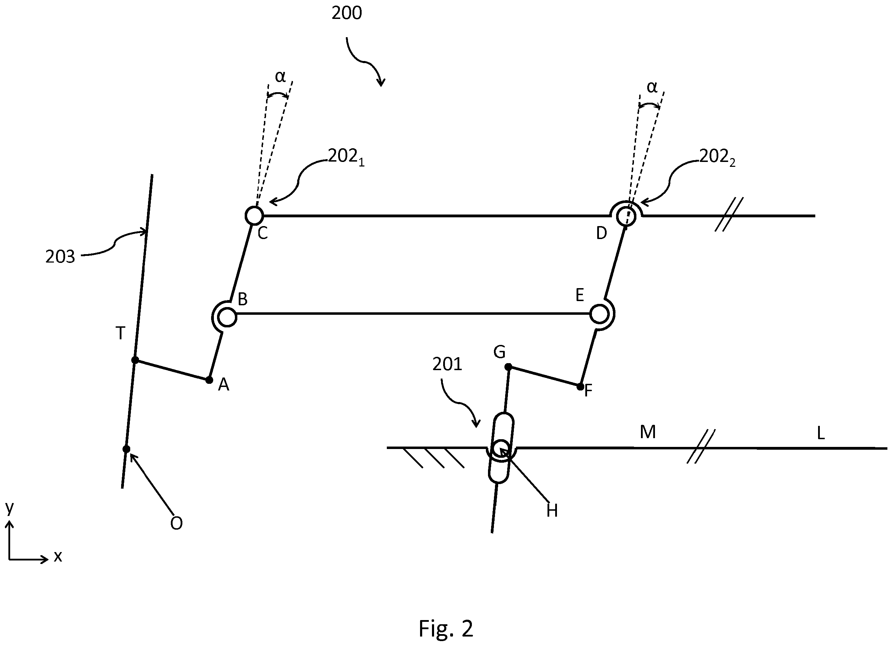

Referring to Figure 2, a second apparatus 200 according to embodiments of the present invention is similar to the first apparatus 100. The second apparatus 200 differs from the first apparatus 100 in that the second apparatus 200 comprises a connecting link AT which is fixed to the tool holder 203 at a first point of fixation T and is fixed to the distal link ABC at a second point of fixation A. The first point of fixation T and the second point of fixation A are spaced apart in a direction transverse to the tool axis OT. The connecting link AT allows further spacing apart of the tool holder 203 from the distal link ABC, providing more free space for accessing the tool holder 203.

The second apparatus 200 further comprises a rigid link FG coupled to an end F of the proximal link DEF. Thus the point at which the joint 201 is coupled to the link DEF is spaced apart from point F in a direction transverse to the tool axis OT. The rigid link FG is advantageously maintained parallel to the connecting link AT, but generally need not be parallel to it.

The angle α is again shown, being the angle between the distal link ABC and the tool axis OT, which is identical to the angle between the proximal link DEF and a line connecting point G with the remote centre of motion H.

Preferably the first motion mechanism is a planar motion mechanism defining a plane of motion comprising the local centre of motion H and the remote centre of motion O. For example, referring still to Figure 2, the first motion mechanism defines a plane of motion which is perpendicular to the z axis. The first motion mechanism is capable of motion within the x-y plane. The revolute joint and the sliding joint 201 are then preferably planar motion joints, such that the proximal link DEF is configured to move in the plane of motion.

An axis of translation can be defined by the direction in which the sliding joint is configured to implement translation. This axis of translation is parallel to the tool axis OT and is oriented at the angle alpha with respect to the orientation of the proximal link DEF. In some embodiments, the proximal link DEF defines a second orientation which is inclined relative to the axis of translation. In such embodiments, the first orientation and the second orientation may be parallel.

In embodiments wherein the apparatus comprises the rigid link BE, the first orientation can be defined by an axis between the pivot joint at C between the distal link ABC and the first intermediate link CD, and the pivot joint at B between the distal link ABC and the second intermediate link BE. Optionally, the second orientation can be defined by an axis between the pivot joint at D between the proximal link DEF and the first intermediate link CD, and the pivot joint at E between the proximal link DEF and the second intermediate link BE. Preferably, in embodiments wherein fixation points A and T are spaced apart, the second point of fixation A is located between the pivot joint at B between the distal link ABC and the second intermediate link BE and the remote centre of motion O.

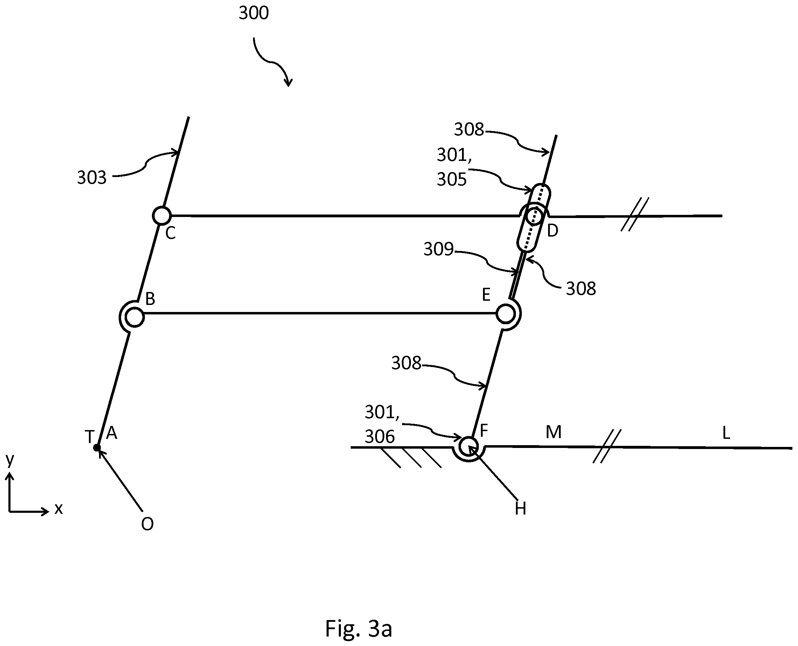

Referring to Figure 3a, a third apparatus 300 according to embodiments of the present invention is shown. The third apparatus 300 comprises the proximal link DEF, the distal link ABC and the first intermediate link CD, the rigid link BE, the base HML, and the tool holder 303. In the third apparatus 300, the distal link ABC and the tool holder axis OT are parallel and coaxial. Points A, T, and O coincide. The rigid link BE may be replaced with other mechanical means as described hereinbefore. The rotational and translational joint 301 is disposed at point D and is decoupled into a translational part 305 and a rotational part 306.

The apparatus 300 comprises a first rigid rod 308 being parallel to the distal link ABC, and a second rigid rod 309 between points D and E. The first rigid rod 308 extends through points F, E, and D and beyond point D, and provides a track along which the second rigid rod 309 can be translated by the translational joint 305, such that points D and E maintain the same position relative to each other and the parallelogram BCDE is maintained. During such action, the distance EF changes, as does the distance DF by the same amount and in the same direction. The proximal link DEF is thus provided by the second rigid rod 309 and at least part of the first rigid rod 308.

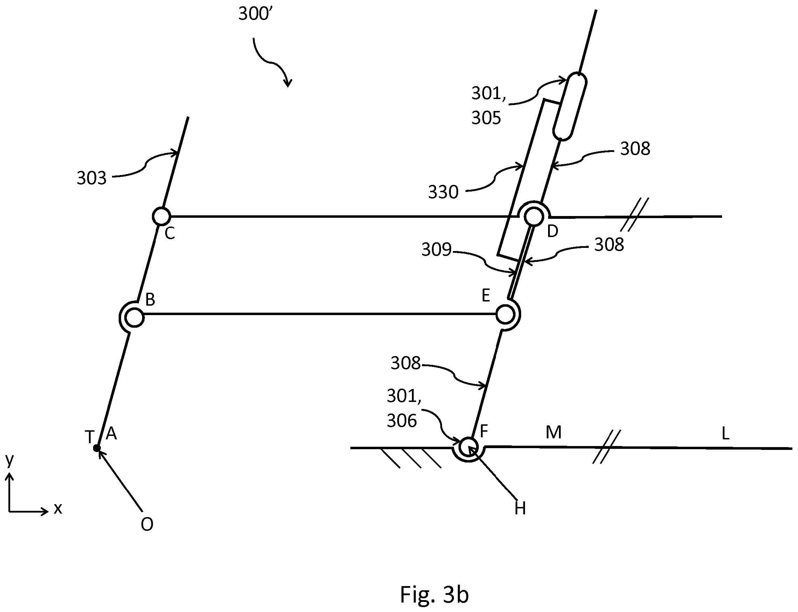

The translational joint can alternatively be provided at point E or between points D and E, or beyond point D and rigidly coupled to the second rigid rod 309 via coupling 330 as shown in apparatus 300' in Figure 3b, or at any point along the axis defined by DEF provided the translational joint is rigidly fixated to the second rigid rod 309 in a collinear manner.

A reference plane can be defined which comprises the remote centre of motion and the local centre of motion, wherein the reference plane is orthogonal to the plane of motion. The sliding joint 305 and the revolute joint 306 are disposed at the same side of the reference plane. This allows the motion of the proximal link DEF to be completely kept on the side of the reference plane where the sliding and rotational joints 305, 306 are disposed, and consequently protruding linkages which may cause injury to a patient or which may cause collision with other devices are eliminated.

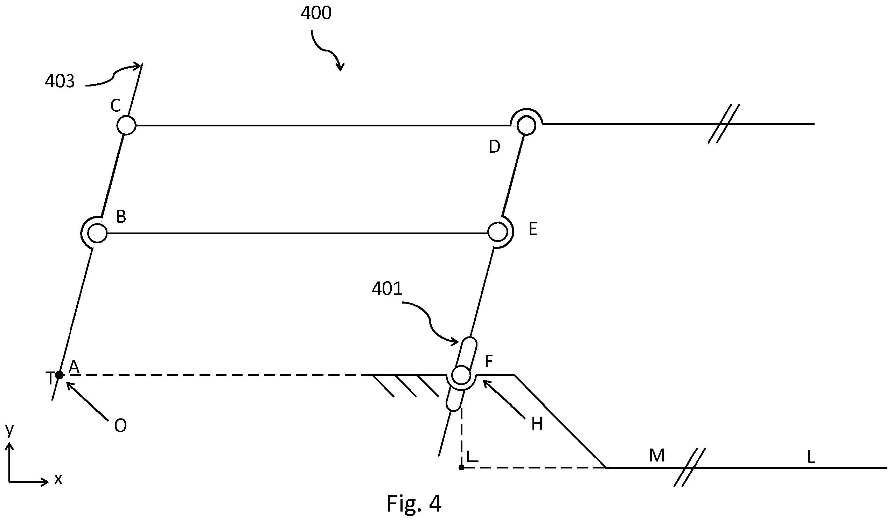

Referring to Figure 4, a fourth apparatus 400 according to embodiments of the present invention is shown. The fourth apparatus 400 comprises the proximal link DEF, the distal link ABC and the first intermediate link CD, the rigid link BE, the base HML, and the tool holder 403. In the fourth apparatus 400, the distal link ABC and the tool holder axis OT are parallel and coaxial. Points A, T, and O coincide. The rigid link BE may be replaced with other mechanical means as described hereinbefore.

The proximal link DEF is coupled to the local centre of motion H through the revolute and sliding joint 401 which allows both rotation about the local centre of motion H and translation relative to the local centre of motion H. The joint 401 is coupled to the proximal link DEF at point G, which in apparatus 400 coincides with point F. In the fourth apparatus 400, joint 401 is a combined prismatic and rotational joint.

In the fourth apparatus 400, the base link HML comprises a segment ML wherein the segment ML is disposed parallel to, and offset from, an axis defined by the local centre of motion H and the remote centre of motion O. This offsetting provides additional space that can be used for at least part of the second motion mechanism, reducing bulkiness of the linkage system which includes both the first and second motion mechanisms. This provides more space for an operator of the apparatus, such as a surgeon, and for other manipulators which may be mounted in the vicinity of the apparatus.

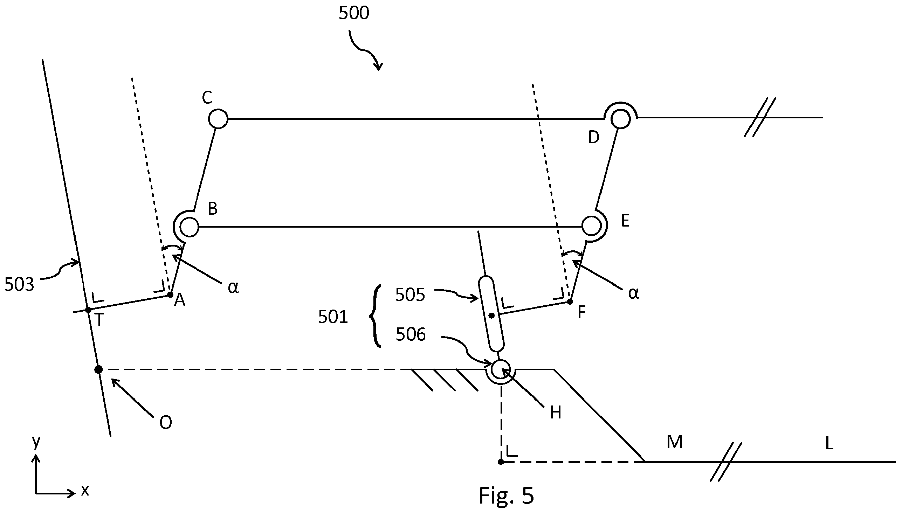

Referring to Figure 5, a fifth apparatus 500 according to embodiments of the present invention is shown. The fifth apparatus 500 comprises the proximal link DEF, the distal link ABC and the first intermediate link CD, the rigid link BE, the base HML, and the tool holder 503.

In the fifth apparatus 500, the tool holder 503 defines a tool axis OT which permanently comprises the remote centre of motion O. The distal link ABC defines a first orientation which is inclined relative to the tool axis OT, as described hereinbefore in relation to Figure 1. The inclination of the distal link ABC with respect to the tool axis OT is shown as angle $\alpha$. The angle $\alpha$ is preferably greater than 0° and less than 90° and is preferably between 5° and 60°, more preferably between 25° and 35°. For example, one preferred value of $\alpha$ is 30°.

In the fifth apparatus 500, the rotational and translational joint 501 is disposed between points F and H and is decoupled into a translational part 505 and a rotational part 506. A reference plane can be defined which comprises the remote centre of motion and the local centre of motion, and a pivot axis of rotational joint 506, wherein the reference plane is orthogonal to the plane of motion. The sliding joint 505 is disposed at the same side of the reference plane as the proximal link DEF. This allows the motion of the proximal link DEF to be completely kept within the perimeter of the other linkages of the apparatus, and protruding linkages which may cause injury to a patient or which may cause collision with other devices are eliminated.

In the fifth apparatus 500, the base link HML comprises a segment ML wherein the segment ML is disposed parallel to, and offset from, an axis defined by the local centre of motion H and the remote centre of motion O. This offsetting provides additional space that can be used for at least part of the second motion mechanism, reducing bulkiness of the linkage system which includes both the first and second motion mechanisms. This provides more space for an operator of the apparatus, such as a surgeon, and for other manipulators which may be mounted in the vicinity of the apparatus.

Therefore the fifth apparatus 500 combines the features of the angular inclination of the tool axis with respect to the distal link, the decoupling of the rotational and translation joint, and the offset of part of the base link. However, it will be understood that each of these features and advantages can be provided in isolation from the other advantages as describes hereinbefore in relation to Figures 1 to 4. It will also be understood that each of these features can be provided in combination with only one of the other features, for example in an apparatus comprising the angular inclination of the tool axis with respect to the distal link and the offset of part of the base link while the rotational and translational joints are not decoupled.

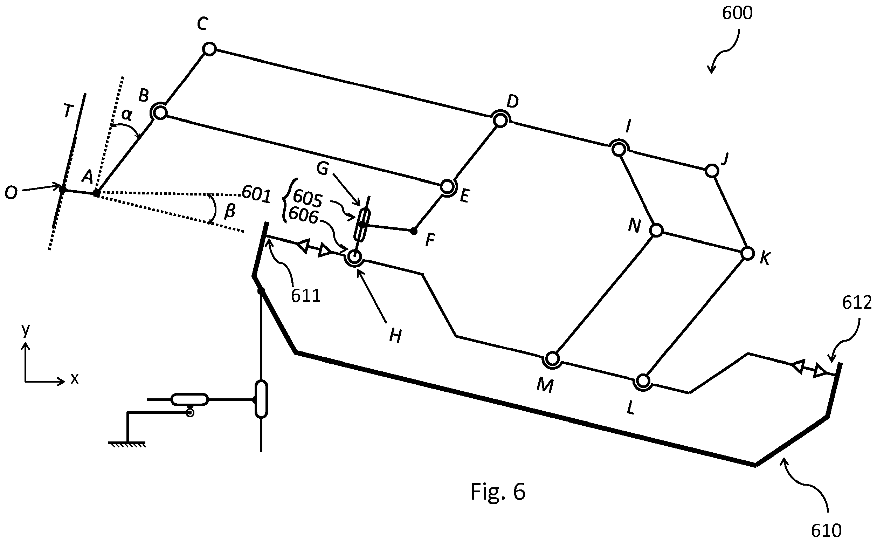

Referring to Figure 6, a sixth apparatus 600 according to embodiments of the present invention is similar to the fifth apparatus 500. The sixth apparatus 600 additionally comprises a second motion mechanism IJNKML which is coupled to the first motion mechanism and is configured to assist motion of the first motion mechanism. The second motion mechanism IJNKML preferably comprises a dual parallelogram arrangement with a first parallelogram IJNK and a second parallelogram NKML. Preferably, the first motion mechanism and the second motion mechanism share a continuous rigid link CDIJ. Put differently, the first intermediate link CD preferably comprises a portion which extends collinearly with CD beyond point D and forms part of the second motion mechanism. The second motion mechanism preferably also comprises rigid links IN, JK, NM, and KL. Rigid link IN is pivotally coupled at a first end at point I to the extended first intermediate link CDIJ and is pivotally coupled at a second end at point N to rigid links NM and NK. Rigid link NM is pivotally coupled at a first end to point N to rigid links IN and NK and is pivotally coupled at a second end at point M to the base link HML. Rigid link JK is pivotally coupled at a first end J to the continuous rigid link CDIJ and is pivotally coupled at a second end K to rigid links NK and KL. Rigid link KL is pivotally coupled at a first end K to rigid links NK and JK and is pivotally coupled at a second end L at point L to the base link HML.

The coupling of the links is such that portions IJ and NK are maintained with an orientation which is parallel to a line connecting the remote and local centres of motion. Links IN and JK are maintained parallel to each other. Links NM and KL are maintained parallel to each other but are not necessarily maintained parallel to the proximal link DEF. Thus the second motion mechanism is preferably configured to maintain at least a segment of the first intermediate link parallel to an axis defined by the local centre of motion and the remote centre of motion.

However, the second motion mechanism is not restricted to a dual parallelogram arrangement and may comprise, for example, a combination of two linear guides or 1 DOF translational joints maintained in an orthogonal configuration, or a serial combination of two parallel belt drives, or other appropriate mechanical means.

The sixth apparatus 600 additionally comprises a support 610. The base link HML is pivotally connected to the support 610 at coupling points 611, 612 respectively. The pivotal connection allows the base link HML to pivot about an axis defined by the remote centre of motion O and the local centre of motion H.

It will be understood that the support 610 can be provided in conjunction with any of the apparatuses described hereinbefore and is not restricted to the sixth apparatus 600. It will be understood that the apparatus 600 need not be provided with the support 610 allowing pivotal movement of the base link HML.

As described hereinbefore, the first motion mechanism may be a planar motion mechanism defining a plane of motion comprising the local and remote centres of motion. In such embodiments wherein the offset arrangement as described in relation to Figure 5 is implemented, the second motion mechanism may be coupled to segment ML of the base link which is disposed at an opposite side of the reference plane compared to the first intermediate link or the proximal link.

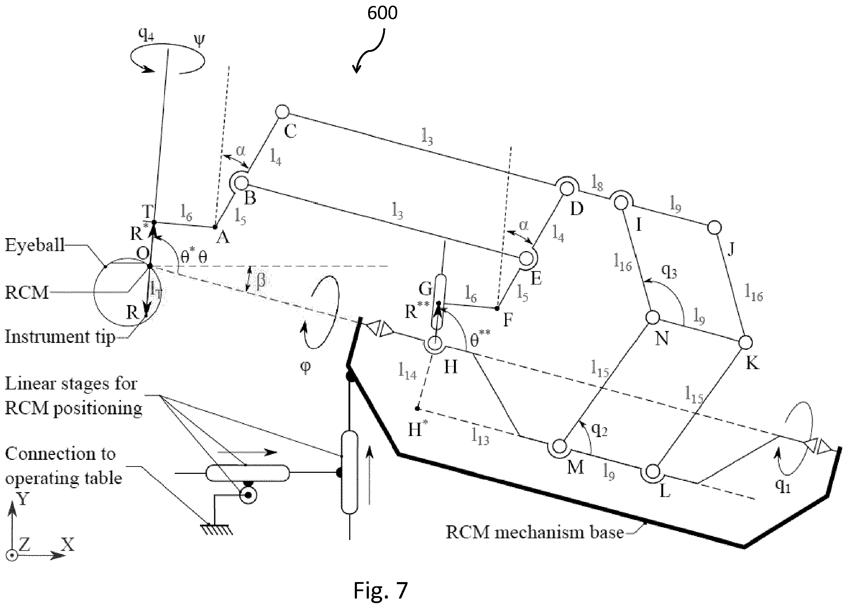

Referring to Figure 7, the sixth apparatus 600 is shown with additional annotations and is now described in more detail. In Figure 7, the sixth apparatus 600 is shown with an instrument coupled to the tool holder.

In Figure 7, the origin of the coordinate system is the remote centre of motion O. The degrees of freedom (DOF) of the remote centre of motion (RCM) are expressed at the instrument tip and are indicated as R, $\theta$, $\varphi$, and $\psi$. Joint DOF are expressed as $q_1$, $q_2$, $q_3$, $q_4$. Additional expressions for the 2 DOF planar kinematics are provided being $R^*$, $\theta^*$ between the RCM and the instrument fixation point T as well as $R^{**}$, $\theta^{**}$ between locations H and G. The lengths of links or link portions are represented as lengths $l_1$, $l_2$, ..., $l_{17}$. Angle $\alpha$ is also shown, defined as described hereinbefore. Instrument length $l_T$ is defined as being measured between the instrument tip and the fixation point T.

The four RCM DOF R, $\theta$, $\varphi$, and $\psi$ defined at the tip of the instrument are realised via a combination of a 2 DOF planar RCM mechanism with two 1 DOF revolute joints. The functioning of the 2 DOF planar mechanism, which provides RCM DOF R and $\theta$, can be understood as follows. A local centre of motion is created through and about H via the combination of a prismatic 805 and a rotary joint 806 as described hereinbefore. The movable part of the prismatic joint is fixated to rigid link GFED at location G, which is expressed as distance $R^{**}$ and angle $\theta^{**}$ with respect to H and the x axis. More specifically, rigid link GFED is composed as follows. A straight link GF is fixated perpendicular to the translational direction of the prismatic joint at location G. GF is rigidly connected to straight link FED at location F, with the angle between GF and FED being $\pi/2 + \alpha$. At locations E and D a rotational joint connects rigid link GFED to rigid links BE and CDIJ respectively. These three rigid links form a parallelogram BCDE with rigid link TABC, which shares an identical geometry with rigid link GFED. In turn, analogous to how rigid link GFED is fixated to the prismatic joint at location G, rigid link TABC holds the instrument at location T. The coordinates of T are expressed as $R^*$ and $\theta^*$ with respect to the origin and the x axis. An RCM is created at the origin through and about which the instrument can move, provided that rigid link CDIJ remains parallel to the x axis. This is achieved via two stacked parallelograms, i.e. a second motion mechanism, IJNK and NKML which connect CDIJ with rigid link HML. ML is thus by design parallel to the x axis.

$R^{**}$ and $\theta^{**}$ are equal to $R^*$ and $\theta^*$ respectively under the condition of the following equalities:

$$l_0 = l_6 \qquad l_1 = l_5 \qquad l_2 = l_4 \qquad l_3 = l_7$$
$$l_9 = l_{17} = l_{12} \qquad l_{10} = l_{16} \qquad l_{11} = l_{15}$$

Consequently, $R^{**}$ and $\theta^{**}$ can then be expressed as RCM DOF R and $\theta$ at the instrument tip by taking into account the instrument length $l_T$. In addition to $R$ and $\theta$ provided by the 2 DOF planar mechanism, the third RCM DOF $\varphi$ is implemented as a 1 DOF revolute joint, the axis of which is collinear with the x axis. As such, the planar mechanism is configured to rotate about the x axis at both ends of the base link HML. The remaining RCM DOF $\psi$ can be implemented as an additional 1 DOF revolute joint about the longitudinal axis of the tool holder at instrument fixation point T.

Joint angles $q_1$ and $q_4$ are implemented by two independent 1 DOF revolute joints implemented in series with the 2 DOF planar RCM mechanism. As such, it can be seen that these are equal to angles $\varphi$ and $\psi$ respectively.

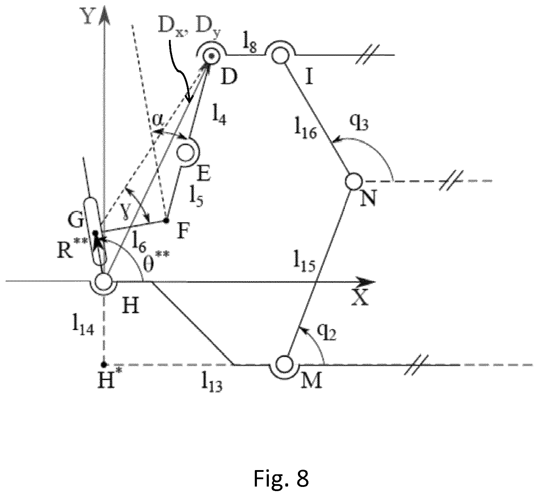

The relation between the joint angles $q_2$, $q_3$ and the end effector variables $R$, $\theta$ can be derived as follows, referring to Figure 8 which shows detail of the planar loop HGFEDINM.

The inverse kinematics of the apparatus describe the apparatus configuration as a function of end-effector variables ($R, \theta, \varphi, \psi$). The resulting apparatus configuration is fully described by the joint angles ($q_1, q_2, q_3, q_4$) according to equation 1:

$$(q_1, q_2, q_3, q_4) = \mathcal{IK}(R, \theta, \phi, \psi) \tag{1}$$

R and $\theta$ can be expressed as a function of $R^{**}$ and $\theta^{**}$ according to equations 2 and 3:

$$R = R^{**} - l_T \tag{2}$$

$$\theta = \theta^{**} \tag{3}$$

Expressing the planar coordinates of D with respect to H as a function of $R^{**}$ and $\theta^{**}$ results in the following relations according to equations 4 and 5:

$$\mathbf{D}_x = R^{**}\cos(\theta^{**}) + l_6\sin(\theta^{**}) + (l_4 + l_5)\cos(\theta^{**} - \alpha) \tag{4}$$

$$\mathbf{D}_y = R^{**}\sin(\theta^{**}) - l_6\cos(\theta^{**}) + (l_4 + l_5)\sin(\theta^{**} - \alpha) \tag{5}$$

Expressing the same planar coordinates as a function of $q_2$ and $q_3$ gives equations 6 and 7:

$$\mathbf{D}_x = l_{15}\cos(q_2) + l_{16}\cos(q_3) + l_{13} - l_8 \tag{6}$$

$$\mathbf{D}_y = l_{15}\sin(q_2) + l_{16}\sin(q_3) - l_{14} \tag{7}$$

From equations 4 to 7 the inverse kinematics as defined in equation 1 can be derived and are expressed according to equations 8 to 11:

$$q_1 = \phi \tag{8}$$

$$q_2 = \operatorname{atan2}\left(\frac{\mathbf{D}_y}{\mathbf{D}_x}\right) \pm \operatorname{acos}\left(\frac{\mathbf{D}_x^2 + \mathbf{D}_y^2 + l_{15}^2 - l_{16}^2}{2l_{15}\sqrt{\mathbf{D}_x^2 + \mathbf{D}_y^2}}\right) \tag{9}$$

$$q_3 = \mp \text{acos}\left(\frac{\mathbf{D}_x^2 + \mathbf{D}_y^2 - l_{15}^2 - l_{16}^2}{2l_{15}l_{16}}\right) + q_2 \qquad (10)$$

$$q_4 = \psi \qquad (11)$$

with $\mathbf{D}_x$ and $\mathbf{D}_y$ expressed as a function of $R^{**}$ and $\theta^{**}$, which in turn can be expressed as a function of $R$ and $\theta$ according to equations 2 and 3.

## Claims

1. Apparatus for generating motion around a remote centre of motion (O), comprising:

   a base link (HML) comprising a local centre of motion (H),
   a first motion mechanism comprising a proximal link (DEF), a distal link (ABC) and a first intermediate link (CD) pivotally interconnecting the proximal link and the distal link, wherein the proximal link is coupled to the local centre of motion through a revolute joint and a sliding joint (101) allowing the proximal link to translate relative to the local centre of motion and to rotate about the local centre of motion, wherein the first motion mechanism is configured to copy motion of the proximal link relative to the local centre of motion to an identical motion of the distal link relative to the remote centre of motion, and
   a tool holder fixed to the distal link (ABC), wherein the tool holder defines a tool axis (OT) intersecting the remote centre of motion (O),

   **characterised in that** the distal link (ABC) defines a first orientation which is inclined relative to the tool axis (OT).

2. Apparatus of claim 1, wherein the first orientation is arranged at an angle ($\alpha$) which is between 5° and 60° relative to the tool axis (OT).

3. Apparatus of claim 1 or 2, comprising a connecting link (AT) rigidly connecting the distal link (ABC) to the tool holder, wherein the connecting link is arranged transverse to the tool axis.

4. Apparatus of the preceding claim, wherein a first point of fixation (T) between the tool holder and the connecting link (AT) and a second point of fixation (A) between the proximal link and the connecting link are spaced apart.

5. Apparatus of any one of the preceding claims, wherein the first motion mechanism is a planar motion mechanism defining a plane of motion comprising the local centre of motion and the remote centre of motion, optionally wherein the revolute joint and the sliding joint are planar motion joints, wherein the proximal link is configured to move in the plane of motion.

6. Apparatus of any one of the preceding claims, wherein the sliding joint (101) defines an axis of translation, wherein the tool axis (OT) is parallel to the axis of translation.

7. Apparatus of claim 6, wherein the proximal link (DEF) defines a second orientation which is inclined relative to the axis of translation, optionally wherein the first orientation and the second orientation are parallel.

8. Apparatus of any one of the preceding claims, wherein the first motion mechanism comprises a second intermediate link (BE), wherein the proximal link, the distal link and the first and second intermediate links are pivotally interconnected to form a parallelogram mechanism.

9. Apparatus of the preceding claim, wherein the first orientation is defined by an axis between a pivot joint (C) between the distal link and the first intermediate link and a pivot joint (B) between the distal link and the second intermediate link, optionally the second orientation is defined by an axis between a pivot joint (D) between the proximal link and the first intermediate link and a pivot joint (E) between the proximal link and the second intermediate link, optionally wherein, in conjunction with claim 4, the second point of fixation (A) is located between a pivot joint (B) between the distal link and the second intermediate link and the remote centre of motion (O).

10. Apparatus of any one of the preceding claims in conjunction with claim 5, wherein the sliding joint (305, 505) is connected to the revolute joint (506), wherein the sliding joint and the proximal link (309) are disposed at a same side of a reference plane comprising the remote centre of motion (O) and the local centre of motion (H), the reference plane being orthogonal to the plane of motion.

11. Apparatus of any one of the preceding claims, comprising a support, wherein the base link (HML) is pivotally connected to a support allowing the base link to pivot about an axis defined by the remote centre of motion (O) and the local centre of motion (H).

12. Apparatus of any one of the preceding claims, comprising a second motion mechanism (IJNKML) coupled to the first motion mechanism and configured to assist motion of the first motion mechanism, optionally wherein the second motion mechanism is coupled between the base link and the first motion mechanism.

13. Apparatus of the preceding claim, wherein the second motion mechanism is coupled to the first intermediate link, the second motion mechanism being configured to maintain at least a segment of the first intermediate link parallel to an axis defined by the local centre of motion and the remote centre of motion.

14. Apparatus of any one of the two preceding claims, wherein the second motion mechanism is coupled to a segment (ML) of the base link disposed parallel to, and offset from, an axis defined by the local centre of motion (H) and the remote centre of motion (O).

15. Apparatus of any one of the three preceding claims, wherein the first motion mechanism is as recited in claim 5, wherein the second motion mechanism is coupled to a segment (ML) of the base link disposed at an opposite side of a reference plane compared to the first intermediate link or the proximal link, wherein the reference plane comprises the remote centre of motion and the local centre of motion, the reference plane being orthogonal to the plane of motion.


**Patentansprüche**

1. Gerät zum Erzeugen von Bewegung um einen Fernbewegungspunkt (O), umfassend:

   einen Basislink (HML), umfassend einen Lokalbewegungspunkt (H),
   einen ersten Bewegungsmechanismus, umfassend einen proximalen Link (DEF), einen distalen Link (ABC) und einen ersten Zwischenlink (CD), der schwenkbar den proximalen Link und den distalen Link miteinander verbindet, wobei der proximale Link mit dem Lokalbewegungspunkt durch ein Drehgelenk und ein Gleitgelenk (101) gekoppelt ist, wodurch ermöglicht wird, der proximale Link relativ zu dem Lokalbewegungspunkt zu verschieben und um den Lokalbewegungspunkt zu drehen, wobei der erste Bewegungsmechanismus konfiguriert ist, um Bewegung des proximalen Links mit Bezug auf den Lokalbewegungspunkt in eine identische Bewegung des distalen Links mit Bezug auf den Fernbewegungspunkt zu kopieren, und
   einen Werkzeughalter, der an den distalen Link (ABC) fixiert ist, wobei der Werkzeughalter eine Werkzeugachse (OT) definiert, die den Fernbewegungspunkt (O) schneidet,
   **dadurch gekennzeichnet, dass** der distale Link (ABC) eine erste Ausrichtung definiert, die mit Bezug auf die Werkzeugachse (OT) geneigt ist.

2. Gerät nach Anspruch 1, wobei die erste Ausrichtung in einem Winkel ($\alpha$) angeordnet ist, der zwischen 5° und 60° mit Bezug auf die Werkzeugachse (OT) beträgt.

3. Gerät nach Anspruch 1 oder 2, umfassend einen Verbindungslink (AT), der starr den distalen Link (ABC) mit dem Werkzeughalter verbindet, wobei der Verbindungslink quer zur Werkzeugachse angeordnet ist.

4. Gerät nach dem vorhergehenden Anspruch, wobei ein erster Fixierungspunkt (T) zwischen dem Werkzeughalter und dem Verbindungslink (AT) und ein zweiter Fixierungspunkt (A) zwischen dem proximalen Link und dem Verbindungslink voneinander beabstandet sind.

5. Gerät nach irgendeinem der vorhergehenden Ansprüche, wobei der erste Bewegungsmechanismus ein planarer Bewegungsmechanismus ist, der eine den Lokalbewegungspunkt und den Fernbewegungspunkt umfassende Bewegungsebene definiert, optional wobei das Drehgelenk und das Gleitgelenk planare Bewegungsgelenke sind, wobei der proximale Link konfiguriert ist, um in der Bewegungsebene zu bewegen.

**6.** Gerät nach irgendeinem der vorhergehenden Ansprüche, wobei das Gleitgelenk (101) eine Translationsachse definiert, wobei die Werkzeugachse (OT) parallel zur Translationsachse ist.

**7.** Gerät nach Anspruch 6, wobei der proximale Link (DEF) eine zweite Ausrichtung definiert, die mit Bezug auf die Translationsachse geneigt ist, optional wobei die erste Ausrichtung und die zweite Ausrichtung parallel sind.

**8.** Gerät nach irgendeinem der vorhergehenden Ansprüche, wobei der erste Bewegungsmechanismus einen zweiten Zwischenlink (BE) umfasst, wobei der proximale Link, der distale Link und der erste und zweite Zwischenlink schwenkbar miteinander verbunden sind, um einen Parallelogrammmechanismus zu bilden.

**9.** Gerät nach dem vorhergehenden Anspruch, wobei die erste Ausrichtung durch eine Achse zwischen einem Drehgelenk (C) zwischen dem distalen Link und dem ersten Zwischenlink und ein Drehgelenk (B) zwischen dem distalen Link und dem zweiten Zwischenlink definiert ist, optional die zweite Ausrichtung durch eine Achse zwischen einem Drehgelenk (D) zwischen dem proximalen Link und dem ersten Zwischenlink und einem Drehgelenk (E) zwischen dem proximalen Link und dem zweiten Zwischenlink definiert ist, optional wobei, in Verbindung mit Anspruch 4, sich der zweite Fixierungspunkt (A) zwischen einem Drehgelenk (B) zwischen dem distalen Link und dem zweiten Zwischenlink und den Fernbewegungspunkt (O) befindet.

**10.** Gerät nach irgendeinem der vorhergehenden Ansprüche in Verbindung mit Anspruch 5, wobei das Gleitgelenk (305, 505) mit dem Drehgelenk (506) verbunden ist, wobei das Gleitgelenk und der proximale Link (309) auf einer gleichen Seite einer den Fernbewegungspunkt (O) und den Lokalbewegungspunkt (H) umfassenden Referenzebene angeordnet sind, wobei die Referenzebene orthogonal zur Bewegungsebene ist.

**11.** Gerät nach irgendeinem der vorhergehenden Ansprüche, umfassend einen Träger, wobei der Basislink (HML) schwenkbar mit einem Träger verbunden ist, der dem Basislink ermöglicht, um eine Achse zu schwenken, die durch den Fernbewegungspunkt (O) und den Lokalbewegungspunkt (H) definiert ist.

**12.** Gerät nach irgendeinem der vorhergehenden Ansprüche, umfassend einen zweiten Bewegungsmechanismus (IJNKML), der mit dem ersten Bewegungsmechanismus gekoppelt ist und konfiguriert ist, um die Bewegung des ersten Bewegungsmechanismus zu unterstützen, optional wobei der zweite Bewegungsmechanismus zwischen dem Basislink und dem ersten Bewegungsmechanismus gekoppelt ist.

**13.** Gerät nach dem vorhergehenden Anspruch, wobei der zweite Bewegungsmechanismus mit dem ersten Zwischenlink gekoppelt ist, wobei der zweite Bewegungsmechanismus konfiguriert ist, um mindestens ein Segment des ersten Zwischenlinks parallel zu einer Achse, die durch den Lokalbewegungspunkt und den Fernbewegungspunkt definiert ist zu halten.

**14.** Gerät nach irgendeinem der zwei vorhergehenden Ansprüche, wobei der zweite Bewegungsmechanismus mit einem Segment (ML) des Basislinks gekoppelt ist, der parallel zu und versetzt von einer Achse, die durch den Lokalbewegungspunkt (H) und den Fernbewegungspunkt (O) definiert ist angeordnet ist.

**15.** Gerät nach irgendeinem der drei vorhergehenden Ansprüche, wobei der erste Bewegungsmechanismus wie in Anspruch 5 angegeben ist, wobei der zweite Bewegungsmechanismus mit einem Segment (ML) des Basislinks gekoppelt ist, der auf einer gegenüberliegenden Seite einer Referenzebene angeordnet ist, verglichen mit dem ersten Zwischenlink oder dem proximalen Link, wobei die Referenzebene den Fernbewegungspunkt und den Lokalbewegungspunkt umfasst, wobei die Referenzebene orthogonal zur Bewegungsebene ist.

**Revendications**

**1.** Appareil de génération de mouvement autour d'un centre distant de mouvement (O), comprenant :

une liaison de base (HML) comprenant un centre local de mouvement (H),
un premier mécanisme de mouvement comprenant une liaison proximale (DEF), une liaison distale (ABC) et une première liaison intermédiaire (CD) reliant entre elles de manière pivotante la liaison proximale et la liaison distale, dans lequel la liaison proximale est couplée au centre local de mouvement par une articulation de rotation et une articulation prismatique (101) permettant à la liaison proximale de se déplacer par rapport au centre local de mouvement et de tourner autour du centre local de mouvement, dans lequel le premier mécanisme de mouvement est configuré pour copier le mouvement de la liaison proximale par rapport au centre local de mouvement sur un mouvement identique de la liaison distale par rapport au centre distant de mouvement, et un porte-outil fixé à la liaison distale (ABC), dans lequel le porte-outil définit un axe d'outil (OT) croisant le centre distant de mouvement (O),

**caractérisé en ce que** la liaison distale (ABC) définit une première orientation qui est inclinée par rapport à l'axe d'outil (OT).

**2.** Appareil selon la revendication 1, dans lequel la première orientation est agencée à un angle ($\alpha$) qui est compris entre 5° et 60° par rapport à l'axe d'outil (OT).

**3.** Appareil selon la revendication 1 ou 2, comprenant une liaison de raccordement (AT) raccordant de manière rigide la liaison distale (ABC) au porte-outil, dans lequel la liaison de raccordement est agencée transversalement à l'axe d'outil.

**4.** Appareil selon la revendication précédente, dans lequel un premier point de fixation (T) entre le porte-outil et la liaison de raccordement (AT) et un second point de fixation (A) entre la liaison proximale et la liaison de raccordement sont espacés.

**5.** Appareil selon l'une quelconque des revendications précédentes, dans lequel le premier mécanisme de mouvement est un mécanisme de mouvement planaire définissant un plan de mouvement comprenant le centre local de mouvement et le centre distant de mouvement, éventuellement dans lequel l'articulation de rotation et l'articulation prismatique sont des articulations de mouvement planaire, dans lequel la liaison proximale est configurée pour se déplacer dans le plan de mouvement.

**6.** Appareil selon l'une quelconque des revendications précédentes, dans lequel l'articulation prismatique (101) définit un axe de translation, dans lequel l'axe d'outil (OT) est parallèle à l'axe de translation.

**7.** Appareil selon la revendication 6, dans lequel la liaison proximale (DEF) définit une seconde orientation qui est inclinée par rapport à l'axe de translation, éventuellement dans lequel la première orientation et la seconde orientation sont parallèles.

**8.** Appareil selon l'une quelconque des revendications précédentes, dans lequel le premier mécanisme de mouvement comprend une seconde liaison intermédiaire (BE), dans lequel la liaison proximale, la liaison distale et les première et seconde liaisons intermédiaires sont raccordées entre elles de manière pivotante pour former un mécanisme en parallélogramme.

**9.** Appareil selon la revendication précédente, dans lequel la première orientation est définie par un axe entre une articulation pivot (C) entre la liaison distale et la première liaison intermédiaire et une articulation pivot (B) entre la liaison distale et la seconde liaison intermédiaire, éventuellement la seconde orientation est définie par un axe entre une articulation pivot (D) entre la liaison proximale et la première liaison intermédiaire et une articulation pivot (E) entre la liaison proximale et la seconde liaison intermédiaire, éventuellement dans lequel, en conjugaison avec la revendication 4, le second point de fixation (A) est situé entre une articulation pivot (B) entre la liaison distale et la seconde liaison intermédiaire et le centre distant de mouvement (O).

**10.** Appareil selon l'une quelconque des revendications précédentes en conjugaison avec la revendication 5, dans lequel l'articulation prismatique (305, 505) est raccordée à l'articulation de rotation (506), dans lequel l'articulation prismatique et la liaison proximale (309) sont disposées sur un même côté d'un plan de référence comprenant le centre distant de mouvement (O) et le centre local de mouvement (H), le plan de référence étant orthogonal au plan de mouvement.

**11.** Appareil selon l'une quelconque des revendications précédentes, comprenant un support, dans lequel la liaison de base (HML) est raccordée de manière pivotante à un support permettant à la liaison de base de pivoter autour d'un axe défini par le centre distant de mouvement (O) et le centre local de mouvement (H).

**12.** Appareil selon l'une quelconque des revendications précédentes, comprenant un second mécanisme de mouvement (IJNKML) couplé au premier mécanisme de mouvement et configuré pour assister le mouvement du premier mécanisme de mouvement, éventuellement dans lequel le second mécanisme de mouvement est couplé entre la liaison de base et le premier mécanisme de mouvement.

**13.** Appareil selon la revendication précédente, dans lequel le second mécanisme de mouvement est couplé à la première liaison intermédiaire, le second mécanisme de mouvement étant configuré pour maintenir au moins un segment de la première liaison intermédiaire parallèle à un axe défini par le centre local de mouvement et le centre distant de mouvement.

**14.** Appareil selon l'une quelconque des deux revendications précédentes, dans lequel le second mécanisme de mouvement est couplé à un segment (ML) de la liaison de base disposé parallèlement, et en décalage par rapport, à un axe défini par le centre local de mouvement (H) et le centre distant de mouvement (O).

**15.** Appareil selon l'une quelconque des trois revendications précédentes, dans lequel le premier mécanisme de mouvement est tel qu'énoncé dans la revendication 5, dans lequel le second mécanisme de mouvement est couplé à un segment (ML) de la liaison de base disposé au niveau d'un côté opposé d'un plan de référence comparativement à la première liaison intermédiaire ou à la liaison proximale, dans lequel le plan de référence comprend le centre distant de mouvement et le centre local de mouvement, le plan de référence étant orthogonal au plan de mouvement.

Fig. 1

Fig. 2

EP 3 831 543 B1

Fig. 3a

Fig. 3b

Fig. 4

EP 3 831 543 B1

Fig. 5

Fig. 6

Fig. 7

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150351857 A